(19) Europäisches Patentamt
European Patent Office
Office européen des brevets

(11) Numéro de publication: **0 494 817 B1**

(12) # FASCICULE DE BREVET EUROPEEN

(45) Date de publication du fascicule du brevet: **06.09.95**

(51) Int. Cl.$^6$: **C07D 261/20**, C07D 275/04, A61K 31/42, A61K 31/425

(21) Numéro de dépôt: **92400033.4**

(22) Date de dépôt: **08.01.92**

(54) **Dérivés de benzisoxazole et de benzisothiazole, leur procédé de préparation et les compositions pharmaceutiques les renfermant.**

(30) Priorité: **08.01.91 FR 9100135**

(43) Date de publication de la demande:
**15.07.92 Bulletin 92/29**

(45) Mention de la délivrance du brevet:
**06.09.95 Bulletin 95/36**

(84) Etats contractants désignés:
**AT BE CH DE DK ES FR GB GR IT LI LU NL PT SE**

(56) Documents cités:
**EP-A- 0 196 096**
**DE-A- 3 247 530**
**GB-A- 2 163 432**

(73) Titulaire: **ADIR ET COMPAGNIE**
**1 rue Carle Hébert**
**F-92415 Courbevoie Cédex (FR)**

(72) Inventeur: **Peglion, Jean-Louis**
**5 Allée des Bégonias**
**F-78110 Le Vesinet (FR)**
Inventeur: **Vian, Joel**
**12 Avenue Sainte Marie**
**F-92370 Chaville (FR)**

(74) Mandataire: **Reverbori, Marcelle**
**ADIR**
**1, rue Carle Hébert**
**F-92415 Courbevoie Cédex (FR)**

**EP 0 494 817 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des dérivés de benzisoxazole et de benzisothiazole, leur procédé de préparation et les compositions pharmaceutiques les renfermant.

Elle concerne particulièrement les dérivés de formule générale I :

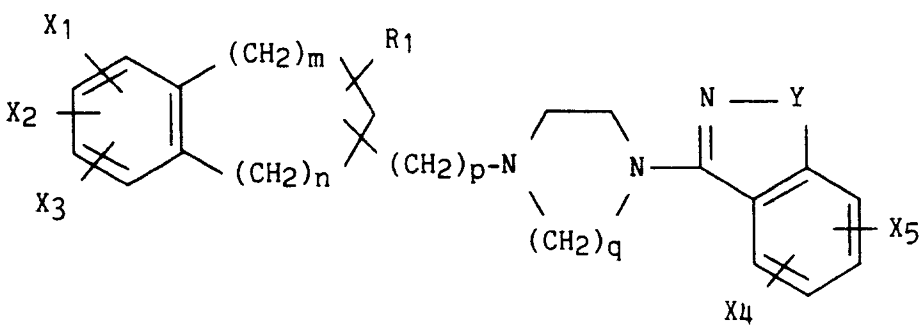

(I)

dans laquelle :

- $X_1$, $X_2$, $X_3$, $X_4$ et $X_5$ :
  - identiques ou différents représentent chacun : un atome d'hydrogène, un atome d'halogène, un radical alkyle, alkoxy ou alkythio dans chacun desquels la partie alkyle renferme de 1 à 5 atomes de carbone en chaine droite ou ramifiée, un radical hydroxy, un radical acyloxy, un radical trifluorométhyle, un radical nitro, un radical amino ou un radical acétamido, ou
  - deux d'entre eux pris en position adjacente forment ensemble un radical méthylènedioxy, un radical éthylènedioxy ou un radical vinylènedioxy ;
- $R_1$ représente un atome d'hydrogène ou un radical alkyle en chaine droite ou ramifiée contenant de 1 à 5 atomes de carbone ;
- m et n représentent chacun les valeurs zéro, un, deux ou trois à condition que $m + n$ soit $\geqq 1$ ;
- p représente ou un nombre entier de 1 à 6 ;
- q représente deux ou trois, et
- Y représente un atome d'oxygène ou le groupe $S(O)_z$ dans lequel z prend les valeurs zero, un ou deux ; sous forme racémique et optiquement actives.

L'état antérieur de la technique dans ce domaine est illustré notamment :

a) soit par des publications concernant des 1,2-benzisoxazole ou benzisothiazole substitués en position 3 par un radical 4-pipéridyl lui-même N-substitué ; de tels composés sont décrits comme ayant des propriétés antipsychotiques -cf les demandes de brevet E.P. 196.132, E.P. 314.098 et les brevets US 4.352.811 et 4.812.461- ou des propiétés analgésiques ou neuroleptiques -cf les brevets US 4.469.869, US 4.355.037, la demande de brevet E.P. 080.104 et J.Med. Chem (1985) 28 p. 761-769 ;

b) soit par des publications concernant des 1,2-benzisothiazole substitués en position 3 par un radical N-pipérazinyl lui-même N'-substitué, pour lesquels est mentionnée soit une activité analgésique non opiacée -cf le brevet GB 2 163 432 soit une activité anxiolytique ou antipsychotique -cf notamment les brevets EP 318 983, EP 329 168 et BE 900 555, et les demandes de brevet EP 1 960 096 et DE 3 247 530.

Toutefois, aucun de ces documents ne suggère les produits objets de la présente invention, lesquels constituent une nouvelle classe d'antipsychotiques particulièrement intéressante du fait de la quasi innocuité de ces produits aux doses actives, en ce qui concerne les effets secondaires et notammment les effets extra-pyramidaux ; alors qu'il est connu que les antipsychotiques peuvent provoquer des effets secondaires très importants ce qui limite leur utilisation.

La présente invention a également pour objet le procédé de préparation des dérivés de formule générale I, caractérisé en ce que l'on condense :

- un dérivé de formule générale II :

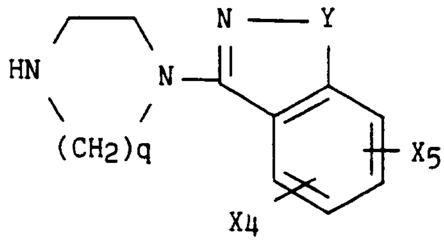

(II)

dans laquelle q, Y, $X_4$ et $X_5$ ont les significations précédemment définies, avec
- un dérivé de formule générale III :

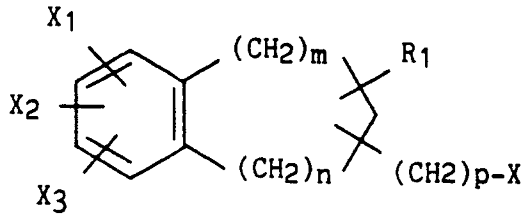

(III)

dans laquelle :
- $X_1$, $X_2$, $X_3$, $R_1$, m, n et p ont les significations précédemment définies et
- X représente un atome d'halogène, un radical mésyloxy ou un radical tosyloxy.

La condensation s'effectue de façon particulièrement adéquate en opérant dans un solvant approprié tel que, par exemple, la méthyléthylcétone, la méthylisobutylcétone, le toluène ou le diméthylformamide en présence d'un accepteur de l'acide formé au cours de la réaction, à une température de 20 à 150 °C. Comme accepteur, on peut employer, par exemple, un carbonate de métaux alcalins comme le carbonate de sodium ou une amine tertiaire comme la triéthylamine.

De plus, les dérivés de formule générale Iont également été préparés selon une variante du procédé précédent, caractérisée en ce que :
- l'on condense :
  - un dérivé de formule générale II précédemment définie, avec,
  - un dérivé de formule générale IV :

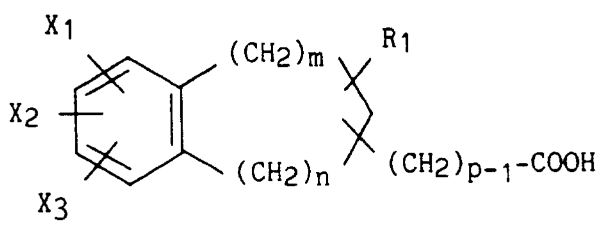

(IV)

dans laquelle $X_1$, $X_2$, $X_3$, $X_4$, $X_5$, $R_1$, m, n et p ont les significations précédemment défines ; et

- l'on réduit l'amide ainsi obtenue de formule générale V :

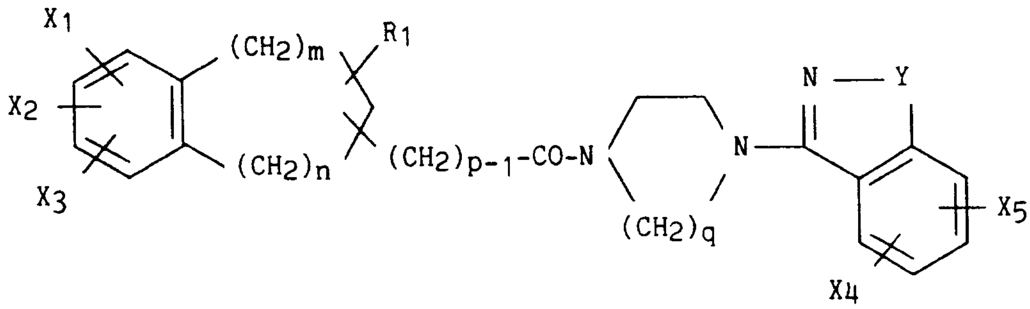

(V)

dans laquelle $X_1$, $X_2$, $X_3$, $X_4$, $X_5$, $R_1$, m, n, p, q et Y ont les significations précédemment définies.

La condensation des dérivés II et IV s'effectue de façon particulièrement adéquate en opérant dans un solvant approprié comme par exemple le chlorure de méthylène, en présence de carbonyldiimidazole.

La réduction de l'amide V s'effectue avantageusement au moyen d'un hydrure double de lithium-aluminium, dans un solvant adéquat, comme par exemple l'éther ou le tétrahydrofurane.

Ce dernier procédé de préparation des dérivés I est également inclus dans la présente invention.

De plus, les amides de formule générale V sont des produits intermédiaires nouveaux qui font, à ce titre, partie de la présente invention.

Les amines de départ de formule générale II ont été préparées par analogie avec la synthèse décrite par J.P. YEVICH et Coll. J. Med Chem. (1986), 29, 359-369.

Les matières premières de formules III et IV sont soit des produits connus, soit des produits préparés à partir de composés connus, selon des procédés connus, comme indiqué dans les exemples ci-après.

Les dérivés de formule générale I donnent des sels avec les acides physiologiquement tolérables. Ces sels sont également inclus dans la présente invention.

Les dérivés de la présente invention possèdent des propriétés pharmacologiques et thérapeutiques intéressantes. En effet, les essais pharmacologiques ont démontré que les dérivés de l'invention sont des antagonistes de la dopamine et de la sérotonine et possèdent une activité antipsychotique comparable à celle de l'halopéridol ou de la chlopromazine, substances de référence pour l'évaluation des antipsychotiques. De plus, aux doses actives, ils n'induisent que peu d'effets secondaires et notamment peu d'effets extra-pyramidaux.

Or il est connu que les antipsychotiques provoquent généralement des effets secondaires très importants, ce qui limite leur utilisation. Les composés de l'invention constituent donc une nouvelle classe d'antipsychotiques utiles pour le traitement de la schizophrénie et celui de la dépression. Ils trouvent aussi leur application comme agents sédatifs, anxiolytiques, anti-agressifs et analgésiques.

La présente invention a également pour objet les compositions pharmaceutiques contenant comme principe actif un dérivé de formule générale I ou un de ses sels physiologiquement tolérables, mélangé ou associé à un excipient pharmaceutique approprié comme par exemple le glucose, le lactose, le talc, l'éthylcellulose, le stéarate de magnésium ou le beurre de cacao.

Les compositions pharmaceutiques ainsi obtenues se présentent généralement sous forme dosée et peuvent contenir de 0,5 à 100 mg de principe actif. Elles peuvent revêtir, par exemple, la forme de comprimés, dragées, gélules, suppositoires, solutions injectables ou buvables et être, selon les cas, administrées par voie orale, rectale ou parentérale à la dose de 0,5 à 100 mg de principe actif 1 à 3 fois par jour.

Les exemples suivants illustrent la présente invention, les points de fusion étant déterminés à la platine chauffante de Kofler sous microscope.

Exemple 1 :

(R,S) 3-{1-[(benzocyclobutan-1-yl)méthyl]pipérazin-4-yl} 6-fluoro 1,2- benzisoxazole

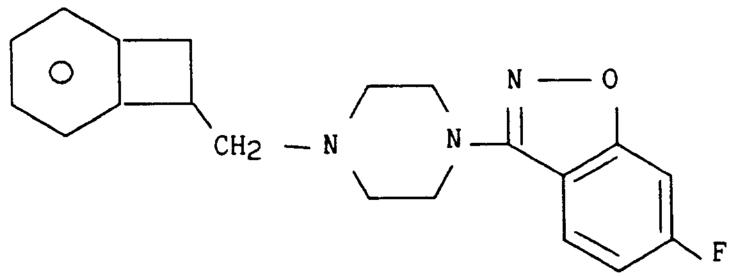

On mélange 4 g ($16.10^{-3}$ mole) d'iodure de (benzocyclobutan-1-yl)méthyle, 3,6 g ($16.10^{-3}$ mole) de N-(6-fluoro 1,2-benzisoxazol-3-yl)pipérazine, 3,5 g ($32.10^{-3}$ mole) de $Na_2CO_3$ et 70 ml de méthylisobutylcétone, et chauffe le tout a 80 °C pendant 12 heures sous agitation. On concentre le mélange réactionnel à l'évaporateur rotatif, reprend le concentrat par l'acétate d'éthyle. Après lavage à l'eau on extrait la phase organique, plusieurs fois, avec une solution normale d'acide chlorhydrique. On alcalinise la phase aqueuse puis l'extrait au $CH_2Cl_2$. Après séchage et chromatographie sur silice en éluant avec le mélange chlorure de méthylène-méthanol, 95-5, on obtient 0,65 g d'un produit solide. On dissout ce dernier dans 10 ml d'éthanol, y ajoute 0,65 ml d'éther chlorhydrique 3N et laisse précipiter durant une nuit. On recueille 0,55 g de chlorhydrate de (R,S) 3-{1-[(benzocyclobutan-1-yl)méthyl]pipérazin-4-yl} 6-fluoro 1,2-benzisoxazole
P.F. : 246-250 °C (avec sublimation vers 190 °C)
Rendement : 9%
RMN (solvant : DMSO d 6)
massif échangeable par $D_2O$ à 11,6 ppm ;
8,15 ppm, 1H (dd) ; 7,6 ppm, 1H (dd) ; 7,1 à 7,3 ppm, 5H (m) ; 4 à 4,3 ppm, 2H (d large) + 1 H (m) ; 3,1 à 3,8 ppm, 10H (m).

L'iodure de (benzocyclobutan-1-yl)méthyle de départ a lui-même été préparé comme suit : 6 g de paratoluènesulfonate de benzocyclobutan-1-yl méthyle [préparé selon le procédé décrit dans JACS (1975), 154, p 347] sont mélangés avec 6,2 g d'iodure de sodium dans 85 ml d'acétone. Le milieu réactionnel est porté à reflux pendant 8 heures puis coulé sur 150 ml d'eau et extrait plusieurs fois à l'éther éthylique. La phase organique est ensuite lavée avec une solution normale de thiosulfate de sodium, séchée sur sulfate de magnésium anhydre et concentrée pour obtenir l'iodure de (benzocyclobutan-1-yl)méthyle, sous forme d'huile .
Rendement : 88%.
Spectre RMN du proton (solvant : $CDCl_3$) :
2,85 ppm d, 1H ; 3,3 à 3,6 ppm m, 3H ; 3,9 ppm, m, 1H ; 7 à 7,3 ppm, m, 4H.

La N(6-fluoro 1,2-benzisoxazol-3-yl)pipérazine de départ a été préparée selon J.P. YEVICH et Coll. J. Med. Chem. (1986) 29 p 359-369.

De la même façon, ont été préparés les dérivés objets des exemples 2 et 3.

Exemples 2 et 3 :

2) Le (R,S) 3-{1-[4-(benzocyclobutan-1-yl)butyl]pipérazin-4-yl} 1,2-benzisothiazole, et son chlorhydrate P.F (instantané) : 202 °C, à partir du bromure de 4-(benzocyclobutan-1-yl)butyle et de N-(1,2-benzisothiazol-3-yl)pipérazine.

3) Le (R,S) 3-{1-[2-(benzocyclobutan-1-yl)éthyl]pipérazin-4-yl} 1,2-benzisothiazole et son chlorhydrate P.F (instantané) : 222 °C, à partir du bromure de 2-(benzocyclobutan-1-yl) éthyle et de N-(1,2-benzisothiazol-3-yl)pipérazine.

Exemple 4 :

(R,S) 3-{1-[(benzocyclobutan-1-yl) méthyl] pipérazin-4-yl} 1,2-benzisothiazole

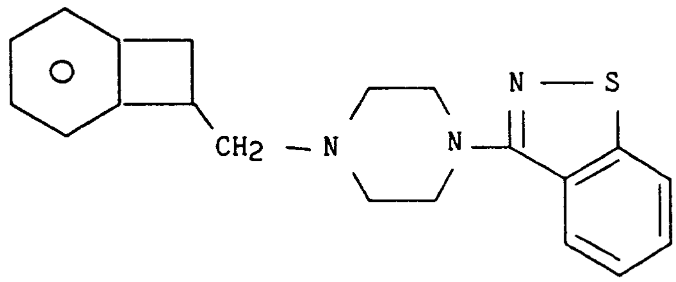

Un mélange de 2,5 g de tosylate de (benzocyclobutan-1-yl)méthyle [préparé selon le procédé décrit dans JACS (1975) 154, p 37], 1,9 g de N-(1,2-benzisothiazol-3-yl)pipérazine[préparée selon J.P.YEVICH et Coll. J. Med. Chem. (1986), 29, p 359-369] et de 1,3 ml de triéthylamine est porté puis maintenu au reflux dans 25 ml de toluène pendant 18 heures. Après refroidissement, on y ajoute 25 ml d'acétate d'éthyle et 20 ml de solution d'hydroxyde de sodium à 20 %. On décante, sèche la phase organique et évapore à sec. Le résidu obtenu est chromatographié sur 300 g de silice en éluant par un mélange de chlorure de méthylène/acétate d'éthyle (90/10). On obtient 1,4 g de produit (Rendement ; 40 %) qui mis dans 5 ml d'acétonitrile puis additionné de 3 ml d'éther chlorhydrique donne le dichlorhydrate de (R,S) 3-{1-[-(benzocyclobutan-1-yl)méthyl]pipérazin-4-yl} 1,2-benzisothiazole, P.F. : 208-215 °C (avec sublimation lente à partir de 170 °C).
RMN (solvant DMSO d6)
8,15 ppm, m, 2H ; 7,4 à 7,6 ppm, m, 2H ; 7 à 7,3 ppm, m, 4H ; 4 à 4,2 ppm, d + m, 3H ; 3,2 à 3,8 ppm, m + d + m + n + m = 9H ; 3,15 ppm, d, 1H ; 11,5 ppm, 2H échangeables.

De la même façon ont été préparés les dérivés objets des exemples 5 à 16.

Exemples 5 à 13 :

**5)** Le (R,S) 3-{1-[(benzocyclobutan-1-yl)méthyl] homopipérazin-4-yl} 1,2-benzisothiazole et son di-(dibenzoyltartrate) P.F. : 110-115 °C, (Rendement : 52,7 %), à partir du tosylate de (benzocyclobutan-1-yl) méthyle et de la N-(1,2 benzisothiazol-3-yl) homopipérazine [elle-même preparée selon J.P. YEVICH et Coll. J. Med. Chem. (1986), 29, 359-369].

**6)** Le (R,S) 3-{1-[(benzocyclobutan-1-yl) méthyl] pipérazin-4-yl} 1,2-benzisoxazole et son chlorhydrate P.F. : 239-243 °C (avec sublimation à partir de 190 °C) (Rendement : 11 %), à partir du tosylate de (benzocyclobutan-1-yl)méthyle et de la N-(1,2-benzisoxazol-3-yl) pipérazine.

**7)** Le (R,S) 3-{1-[(indan-1-yl) méthyl] pipérazin-4-yl} 1,2-benzisothiazole, P.F. : 88-90 °C, (Rendement 30 %), à partir du tosylate d'(indan-1-yl) méthyle et de la N-(1,2-benzisothiazol-3-yl) pipérazine.

**8)** Le (R,S) 3-{1-[(5,6-diméthoxy-indan-1-yl) méthyl] pipérazin-4-yl} 1,2-benzisothiazole, P.F. : 131-133 °C (Rendement 3,6%), à partir du tosylate de (5,6-diméthoxy-indan-1-yl) méthyle et de la N-(1,2-benzisothiazol-3-yl) pipérazine.
Le tosylate de (5,6-diméthoxy-indan-1-yl)méthyle de départ a lui-même été préparé comme suit :
A 3,1 g d'hydrure de lithium aluminium en suspension dans 30 ml de tétrahydrofurane sont ajoutés 18 g d'acide (5,6-diméthoxy-indan-1-yl)carboxylique [lui-même décrit dans J. Pharm. Sci. (1968) 57 (6) 1013] dissous dans 180 ml de tétrahydrofurane. Après 18 h d'agitation à température ambiante sont ajoutés goutte à goutte en refroidissant 2,2 ml d'eau, puis 1,7 ml de soude à 20 % et enfin 7,6 ml d'eau. Les sels minéraux sont alors filtrés et rincés et le filtrat est evaporé à sec. On obtient ainsi 16,5 g de (5,6-diméthoxy-indan-1-yl) méthanol, sous forme d'huile, avec un rendement de 98 %.
RMN (solvant : $CDCl_3$) :
6,8 ppm, s, 2H ; 3,9 ppm, s, 6H ; 3,8 ppm, d, 2H ; 3,3 ppm, m, 1H ; 2,9 ppm, m, 2H ; 2,3 ppm, m, 1H ; 2 ppm, m, 1H ; 1,8 ppm, 1H échangeable.

Aux 16,5 g de l'alcool précédemment obtenu dissous dans 100 ml de pyridine, sont ajoutés 23,7 g de p.toluènesulfochlorure. Après 18 heures d'agitation à température ambiante, le milieu réactionnel est concentré sous vide . Le résidu obtenu est repris par du chlorure de méthylène puis lavé à l'acide

sulfurique normal. Après décantation, la phase organique est séchée puis évaporée à sec. On obtient 31 g de tosylate de (5,6-diméthoxy-indan-1-yl) méthyle, sous forme d'huile, avec un rendement de 97,5 %.
RMN (solvant : $CDCl_3$) :
7,7 ppm, d, 2H ; 7,3 ppm, d, 2H ; 6,7 ppm, 2s, 2H ; 3,9 à 4,3 ppm, m, 2H ; 3,8 ppm, 2s, 6H ; 3,5 ppm, m 1H ; 2,8 ppm, m, 2H ; 2,4 ppm, s, 3H ; 2,25 ppm, m, 1H ; 1,8 ppm, m, 1H.

**9)** Le (R,S) 3-{1-[(4,5-diméthoxybenzocyclobutan-1-yl)méthyl]pipérazin-4-yl} 1,2-benzisothiazole et son chlorhydrate P.F>260 °C, à partir du tosylate de (4,5-diméthoxybenzocyclobutan-1-yl)méthyle et de la N-(1,2-benzisothiazol-3-yl) pipérazine.

**10)** Le (R,S) 3-{1-[3-(benzocyclobutan-1-yl)propyl]pipérazin-4-yl} 1,2-benzisothiazole et son chlorhydrate P.F>260 °C, à partir du mésylate de 3-(benzocyclobutan-1-yl)propyle et de la N-(1,2-benzisothiazol-3-yl) pipérazine.

**11)** Le (R,S) 3-{1-[2-(4,5-diméthoxy benzocyclobutan-1-yl)éthyl]pipérazin-4-yl} 1,2-benzisothiazole et son chlorhydrate P.F : 198-200 °C, à partir du tosylate de 2-(4,5-diméthoxybenzocyclobutan-1-yl) éthyle et de la N-(1,2-benzisothiazol-3-yl) pipérazine.

**12)** Le 3-{1-[(5,6-diméthoxyindan-2-yl) méthyl] pipérazin-4-yl} 1,2-benzisothiazole P.F : 102-105 °C, à partir du tosylate de (5,6-diméthoxyindan-2-yl) méthyle et de la N-(1,2-benzisothiazol-3-yl) pipérazine.

**13)** Le 3-{1-[(indan-2-yl) méthyl] pipérazin-4-yl} 1,2-benzisothiazole P.F : 88-90 °C, à partir du tosylate d'(indan-2-yl) méthyle et de la N-(1,2-benzisothiazol-3-yl) pipérazine.

Exemple 14 :

(R,S) 3-{1-[(4,5-diméthoxybenzocyclobutan-1-yl) méthyl] pipérazin-4-yl} 6-fluoro 1,2-benzisoxazole :

a) première étape :

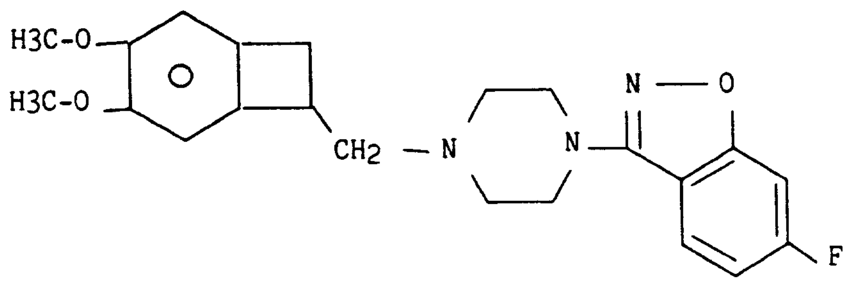

On mélange 2,12 g ($1{,}02.10^{-2}$ mole) d'acide (4,5-diméthoxybenzocyclobutan-1-yl) carboxylique dans 12 ml de chlorure de méthylène et y ajoute, en une seule fois, 1,72 g ($1{,}06.10^{-2}$ mole) de carbonyldiimidazole. On maintient sous agitation pendant une nuit à température ambiante puis ajoute, à la spatule, 2,26 g ($1{,}02.10^{-2}$ mole) de N-(6-fluoro 1,2-benzisoxazol-3-yl) pipérazine [préparée selon J.P. YEVICH et Coll. J. Med. Chem. (1986), 29, p 359-369].

Le mélange réactionnel est laissé à température ambiante pendant 48 heures, puis additionné d'eau. Après décantation, la phase organique est lavée à l'eau puis avec une solution de $NaHCO_3$. Après séchage on obtient une gomme que l'on cristallise dans l'acétate d'éthyle. On obtient alors 2,4 g de (R,S) 3-{1-[(4,5-diméthoxybenzocyclobutan-1-yl) carbonyl] pipérazin-4-yl} 6-fluoro 1,2-benzisoxazole, P.F. : 200 °C,
Rendement : 57 %
RMN (solvant : $CDCl_3$) :
7,65 ppm, 1H (dd) ; 7,15 ppm, 1H (dd) ; 7,05 ppm, 1H (td) ; 6,7 à 6,8 ppm, 2H (2s) ; 4,4 ppm, 1H (t) ; 3,7 à 4 ppm, 4H (m) + 6H (2s) ; 3,65 ppm, 4H (m) ; 3,4 ppm, 2H (d).

L'acide (4,5-diméthoxybenzocyclobutan-1-yl) carboxylique de départ a été préparé selon le procédé décrit dans Tétrahedron (1973), 29, p 73.

b) deuxième étape :

Sous atmosphère d'azote, on ajoute 0,26 g ($7.10^{-3}$ mole) d'hydrure de lithium-aluminium à 41 ml de tétrahydrofurane anhydre : on amène l'ensemble à 30 °C puis ajoute, à la spatule, 2,4 g ($5{,}8.10^{-3}$ mole) de (R,S) 3-{1-[(4,5-diméthoxy benzocyclobutan-1-yl) carbonyl] pipérazin-4-yl} 6-fluoro 1,2-benzisoxazole, précédemment obtenu. On maintient la température du mélange réactionnel à 30 °C pendant 8 heures. Puis on refroidit l'ensemble et l'hydrolyse avec 0,9 ml d'eau et 0,15 ml de solution d'hydroxyde de

sodium à 20 %. Après filtration des sels et décantation du mélange réactionnel, on lave la phase organique à l'eau puis la sèche sur $MgSO_4$. Après chromatographie sur silice en éluant avec le mélange chlorure de méthylène/acétate d'éthyle (90/10), puis recristallisation dans 4,5 ml d'acétate d'éthyle, on obtient 0,63 g de (R,S) 3-{1-[(4,5-diméthoxybenzocyclobutan-1-yl) méthyl] pipérazin-4-yl} 6-fluoro 1,2-benzisoxazole, P.F. : 141-143 ° C, Rendement : 27 %.
RMN (solvant : $CDCl_3$) :
7,65 ppm, 1H (dd) ; 7,15 ppm, 1H (dd) ; 6,95 ppm, 1H (td) ; 6,7 ppm, 2H (2s) ; 3,85 ppm, 6H (s) ; 3,6 ppm, 5H (m) ; 3,3 ppm, 1H (dd) ; 2,6 à 3 ppm, 1H (dd) + 2H (m) + 4H (m).

De la même façon ont été préparés les dérivés objets des exemples 18 à 17.

Exemples 15 - 17 :

**15)** Le (R,S) 3-{1-[(indan-1-yl)méthyl] pipérazin-4-yl} 6-fluoro 1,2-benzisoxazole, P.F. : 109-111 ° C, (rendement : 28 %, à partir de (R,S) 3-{1-[(indan-1-yl)carbonyl] pipérazin-4-yl} 6-fluoro 1,2-benzisoxazole, P.F. : 151 ° C, lui-même préparé, avec un rendement de 55 %, à partir d'acide indan-1-yl carboxylique et de N-(6-fluoro 1,2 benzisoxazol-3-yl) pipérazine.

**16)** Le (R,S) 3-{1-[(4,5-diméthoxybenzocyclobutan-1-yl) méthyl] pipérazin-4-yl} 1,2-benzisoxazole, P.F. : 136-139 ° C, (Rendement : 52 %), à partir de (R,S) 3-{1-[(4,5-diméthoxybenzocyclobutan-1-yl) carbonyl] pipérazin-4-yl} 1,2-benzisoxazole, P.F. : 165 ° C, lui-même préparé, avec un rendement de 80 % à partir d'acide (4,5-diméthoxybenzocyclobutan-1-yl) carboxylique et de N-(1,2-benzisoxazol-3-yl) pipérazine.

**17)** Le (R,S) 3-{1-[(indan-1-yl) méthyl] pipérazin-4-yl} 1,2-benzisoxazole, P.F. : 83-86 ° C, (Rendement : 44 %), à partir de (R,S) 3-{1-[(indan-1-yl) carbonyl] pipérazin-4-yl} 1,2-benzisoxazole, P.F. : 114 ° C, lui-même préparé, avec un rendement de 80 %, à partir d'acide indan-1-yl carboxylique et de N-(1,2-benzisoxazol-3-yl) pipérazine.

Exemple 18 :

**Etude pharmacologique.**

Test des stéréotypies induites par le méthylphénidate chez le rat :

Pour cet essai, on a utilisé 24 rats Wistar mâles, d'un poids compris entre 220 et 240 g, à jeun depuis environ 24 heures. Chaque animal reçoit un premier traitement (composé de référence, dérivé de l'invention ou solvant) à un temps déterminé avant un deuxième traitement (méthylphénidate ou sérum physiologique) effectué par voie I.P. au temps nommé $T_0$. Parmi les animaux utilisés lors d'un essai, quatre servent de contrôle et reçoivent les traitements suivants :
sérum + sérum I.P. : 1 animal (contrôle sérum) ; solvant + méthylphénidate à 40 mg/kg I.P. : 2 animaux (contrôle méthylphénidate à 40 mg/kg I.P.) et solvant + méthylphénidate à 0,16 ; 0,63 ; 2,5 ou 10 mg/kg I.P. : 1 animal (contrôles méthylphénidate 0,16 ; 0,63 ; 2,5 ou 10 mg/kg I.P.).

Les produits étudiés ont été administrés à des temps donnés avant l'injection à $T_0$ de 40 mg/kg I.P. de méthylphénidate. Chaque animal est observé pendant 10 secondes. Les observations comportementales ont eu lieu 30 minutes après le traitement au méthylphénidate. Un total de 10 périodes d'observation est réalisé pour chaque rat au temps $T_{30}$ mn. Pendant ces observations, la présence (1) ou l'absence (0) de stéréotypie est notée.

L'analyse statistique a consisté à comparer, pour une stéréotypie donnée, les taux des scores (0 à 10) obtenus par un groupe d'animaux ayant reçu le même traitement, à ceux obtenus par le groupe d'animaux contrôles méthylphénidate 40 mg/kg, selon le test de Mann et Whitney avec la signification $p = 0,05$ (cf Siegel S et Castelan N.J. 1988).

A titre d'exemple d'évaluation de l'activité antipsychotique des produits de la présente invention, sont répertoriées dans le tableau ci-après les doses efficaces moyennes ($ED_{50}$), c'est à dire les doses permettant une inhibition de 50 % du mâchonnement.

| Produits des exemples n° | ED50 (mg/kg I.P.) |
|---|---|
| 1 | 0,31 |
| 4 | 10 |
| 6 | 1,25 |
| 14 | 2,5 |
| 15 | 10 |
| 16 | 1,25 |
| chlorpromazine | 1,4 |

## Revendications

## Revendications pour les Etats contractants suivants : AT, BE, CH, DE, DK, FR, GB, IT, LI, LU, NL, PT, SE

**1.** Les dérivés de benzisoxazole et de benzisothiazole de formule générale I :

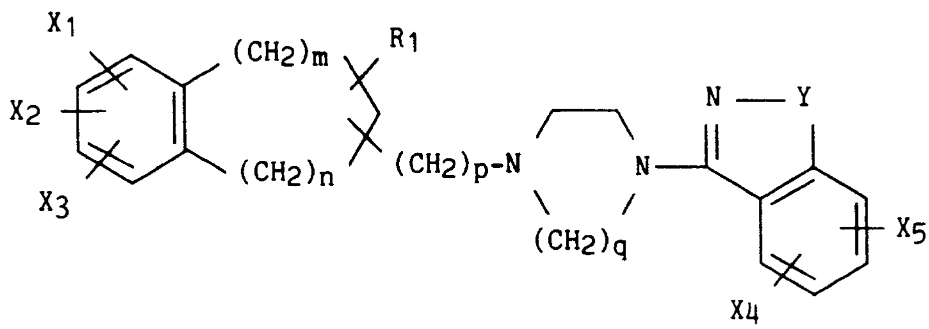

(I)

dans laquelle :

- $X_1$, $X_2$, $X_3$, $X_4$ et $X_5$ :
  - identiques ou différents représentent chacun : un atome d'hydrogène, un atome d'halogène, un radical alkyle, alkoxy ou alkythio dans chacun desquels la partie alkyle renferme de 1 à 5 atomes de carbone en chaine droite ou ramifiée, un radical hydroxy, un radical acyloxy, un radical trifluorométhyle, un radical nitro, un radical amino ou un radical acétamido, ou
  - deux d'entre eux pris en position adjacente forment ensemble un radical méthylènedioxy, un radical éthylènedioxy ou un radical vinylènedioxy ;
- $R_1$ représente un atome d'hydrogène ou un radical alkyle en chaine droite ou ramifiée contenant de 1 à 5 atomes de carbone ;
- m et n représentent chacun les valeurs zéro, un, deux ou trois à condition que $m + n$ soit $\geqq 1$ ;
- p représente un nombre entier de 1 à 6 ;
- q représente deux ou trois, et
- Y représente un atome d'oxygène ou le groupe $S(O)_z$ dans lequel z prend les valeurs zéro, un ou deux ; sous forme racémique et optiquement actives.

**2.** Les sels physiologiquement tolérables des dérivés de la revendication 1 avec des acides appropriés.

**3.** Un dérivé de la revendication 1 qui est le (R,S) 3-{1-[(benzocyclobutan-1-yl) méthyl] pipérazin-4-yl} 6-fluoro 1,2-benzisoxazole, et son chlorhydrate.

**4.** Un dérivé de la revendication 1 qui est le (R,S) 3-{1-[4-(benzocyclobutan-1-yl)butyl]pipérazin-4-yl}1,2-benzisothiazole et son chlorhydrate.

**5.** Un dérivé de la revendication 1 qui est le (R,S) 3-{1-[(benzocyclobutan-1-yl) méthyl] pipérazin-4-yl} 1,2-benzisoxazole, et son chlorhydrate.

**6.** Un dérivé de la revendication 1 qui est le (R,S) 3-{1-[3-(benzocyclobutan-1-yl)propyl] pipérazin-4-yl} 1,2-benzisothiazole et son chlorhydrate.

7. Un dérivé de la revendication 1 qui est le 3-{1-[(5,6-diméthoxyindan-2-yl) méthyl] pipérazin-4-yl} 1,2-benzisothiazole.

8. Un dérivé de la revendication 1 qui est le (R,S)) 3-{1-[4,5-diméthoxybenzocyclobutan-1-yl)méthyl] pipérazin-4-yl} 6-fluoro 1,2-benzisoxazole.

9. Un dérivé de la revendication 1 qui est le (R,S) 3-{1-[4,5-diméthoxybenzocyclobutan-1-yl)méthyl] pipérazin-4-yl} 1,2-benzisoxazole.

10. Le procédé de préparation des dérivés de la revendication 1 caractérisé en ce que l'on condense :
- un dérivé de formule générale II :

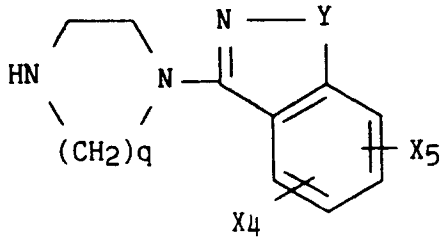

(II)

dans laquelle q, Y, $X_4$ et $X_5$ ont les significations définies dans la revendication 1, avec
- un dérivé de formule générale III :

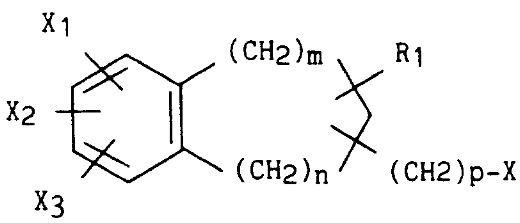

(III)

dans laquelle :
- $X_1$, $X_2$, $X_3$, $R_1$, m, n et p ont les significations définies dans la revendication 1, et
- X représente un atome d'halogène, un radical mésyloxy ou un radical tosyloxy.

11. Le procédé de préparation selon la revendication 10 caractérisé en ce que l'on effectue la condensation des dérivés II et III dans un solvant approprié, à une température comprise entre 20 et 150°C, en présence d'un accepteur de l'acide formé au cours de la réaction.

12. Le procédé de préparation des dérivés de la revendication 1 caractérisé en ce que :
- l'on condense :
  - un dérivé de formule générale II définie dans la revendication 10, avec,
  - un dérivé de formule générale IV :

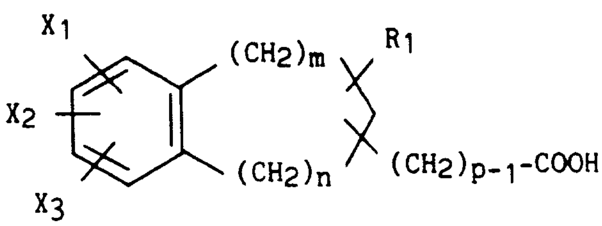

(IV)

dans laquelle $X_1$, $X_2$, $X_3$, $X_4$, $X_5$, $R_1$, m, n et p ont les significations défines dans la revendication 1; et

- l'on réduit l'amide ainsi obtenue de formule générale V :

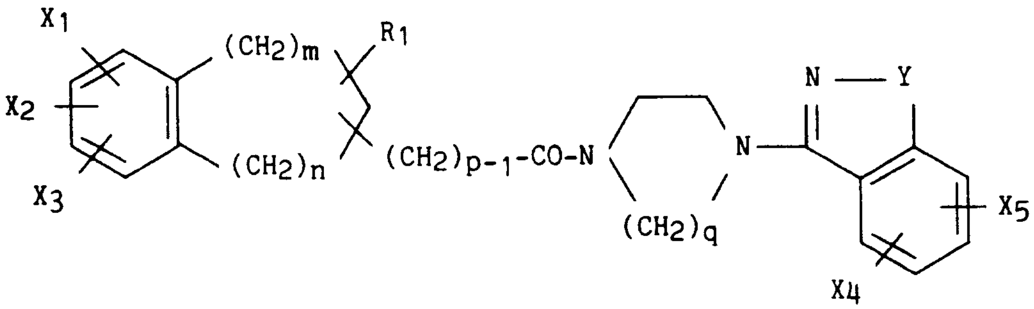

(V)

dans laquelle $X_1$, $X_2$, $X_3$, $X_4$, $X_5$, $R_1$, m, n, p, q et Y ont les significations précédemment définies.

**13.** Le procédé de préparation selon la revendication 12 caractérisé en ce que l'on effectue la condensation des dérivés II et IV dans le chlorure de méthylène en présence de carbonyldiimidazole.

**14.** Le procédé de préparation selon les revendications 12 et 13 caractérisé en ce que l'on effectue la réduction de l'amide V au moyen d'un hydrure double de lithium-aluminium dans un solvant approprié.

**15.** A titre de produits intermédiaires utilisables dans la synthèse des dérivés I selon la revendication 12, les amides de formule générale V :

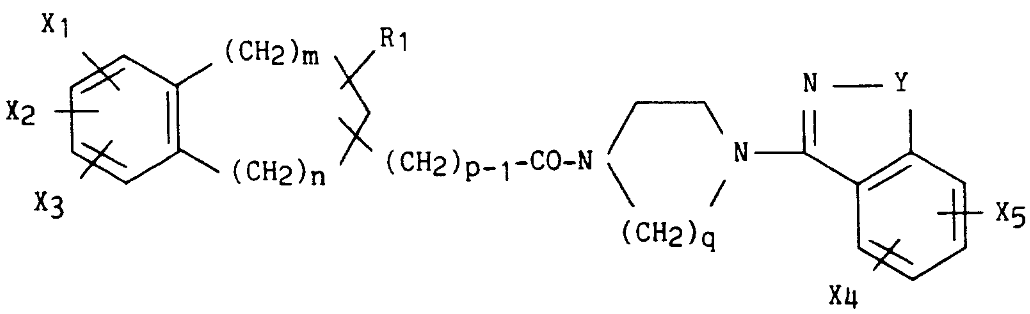

(V)

dans laquelle $X_1$, $X_2$, $X_3$, $X_4$, $X_5$, $R_1$, m, n, p, q et Y ont les significations définies dans la revendication 1.

**16.** Les compositions pharmaceutiques contenant commme principe actif un dérivé selon les revendications 1 à 10 avec des excipients pharmaceutiques appropriés.

**17.** Les compositions pharmaceutiques selon la revendication 16, présentées sous une forme convenant notammment pour le traitement de la douleur, l'anxiété, la psychose, la schizophrénie et la dépression.

**Revendications pour les Etats contractants suivants : ES, GR**

**1.** Le procédé de préparation des dérivés de benzisoxazole et de benzisothiazole de formule générale I :

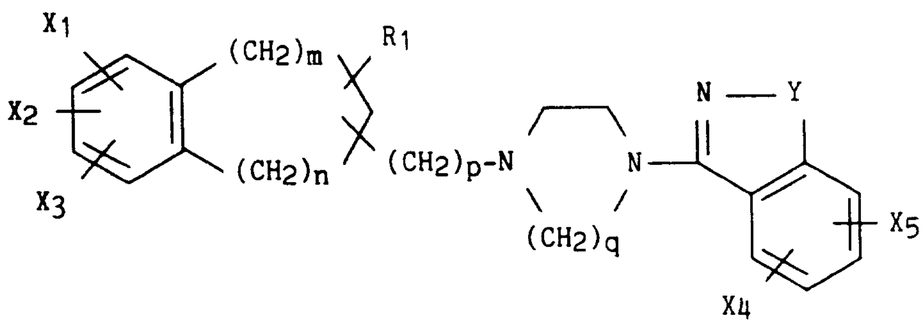

(I)

dans laquelle :

- $X_1$, $X_2$, $X_3$, $X_4$ et $X_5$ :
  - identiques ou différents représentent chacun : un atome d'hydrogène, un atome d'halogène, un radical alkyle, alkoxy ou alkythio dans chacun desquels la partie alkyle renferme de 1 à 5 atomes de carbone en chaine droite ou ramifiée, un radical hydroxy, un radical acyloxy, un radical trifluorométhyle, un radical nitro, un radical amino ou un radical acétamido, ou
  - deux d'entre eux pris en position adjacente forment ensemble un radical méthylènedioxy, un radical éthylènedioxy ou un radical vinylènedioxy ;
- $R_1$ représente un atome d'hydrogène ou un radical alkyle en chaine droite ou ramifiée contenant de 1 à 5 atomes de carbone ;
- m et n représentent chacun les valeurs zéro, un, deux ou trois à condition que $m + n$ soit $\geqq 1$ ;
- p représente un nombre entier de 1 à 6 ;
- q représente deux ou trois, et
- Y représente un atome d'oxygène ou le groupe $S(O)_z$ dans lequel z prend les valeurs zéro, un ou deux ;

  sous forme racémique et optiquement actives, et de leurs sels d'addition acides.physiologiquement tolérables,

  caractérisé en ce que l'on condense :
- un dérivé de formule générale II :

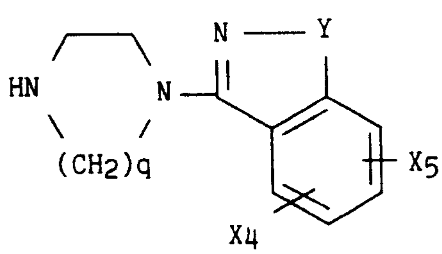

(II)

dans laquelle q, Y, $X_4$ et $X_5$ ont les significations précédemment définies, avec

- un dérivé de formule générale III :

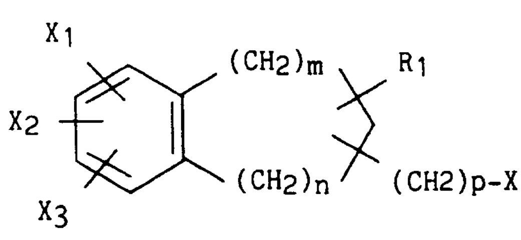

(III)

dans laquelle :

- $X_1$, $X_2$, $X_3$, $R_1$, m, n et p ont les significations précédemment définies , et
- X représente un atome d'halogène, un radical mésyloxy ou un radical tosyloxy ;

et si on le désire, le dérivé ainsi obtenu est traité par des acides appropriés pour obtenir les sels d'addition acides correspondants.

**2.** Le procédé de préparation selon la revendication 1 caractérisé en ce que l'on effectue la condensation des dérivés II et III dans un solvant approprié, à une température comprise entre 20 et 150 °C, en présence d'un accepteur de l'acide formé au cours de la réaction.

**3.** Le procédé de préparation des dérivés de formule I définie dans la revendication 1, caractérisé en ce que :

- l'on condense :
  - un dérivé de formule générale II définie dans la revendication 1, avec,
  - un dérivé de formule générale IV :

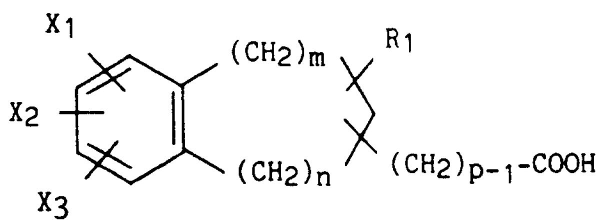

(IV)

dans laquelle $X_1$, $X_2$, $X_3$, $X_4$, $X_5$, $R_1$, m, n et p ont les significations défines dans la revendication 1 ; et

- l'on réduit l'amide ainsi obtenue de formule générale V :

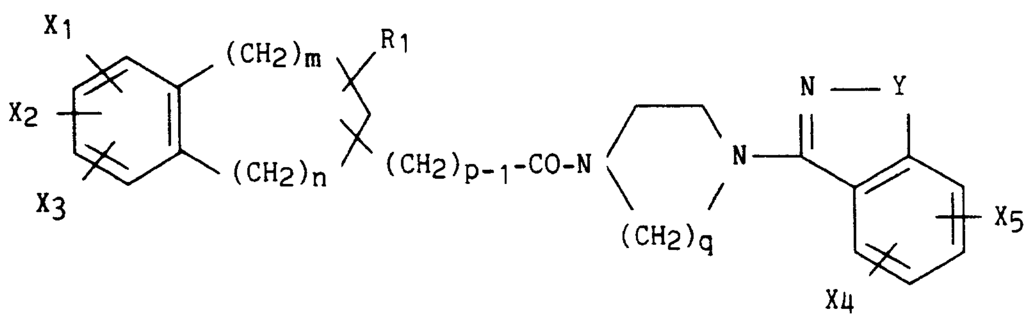

(V)

dans laquelle $X_1$, $X_2$, $X_3$, $X_4$, $X_5$, $R_1$, m, n, p, q et Y ont les significations précédemment définies ; et si on le désire, le dérivé ainsi obtenu est traité par des acides appropriés pour obtenir les sels d'addition acides correspondants.

**4.** Le procédé de préparation selon la revendication 3 caractérisé en ce que l'on effectue la condensation des dérivés II et IV dans le chlorure de méthylène en présence de carbonyldiimidazole.

**5.** Le procédé de préparation selon les revendications 3 et 4 caractérisé en ce que l'on effectue la réduction de l'amide V au moyen d'un hydrure double de lithium-aluminium dans un solvant approprié.

## Claims

**Claims for the following Contracting States : AT, BE, CH, DE, DK, FR, GB, IT, LI, LU, NL, PT, SE**

1. Benzisoxazole and benzisothiazole compounds of the general formula I:

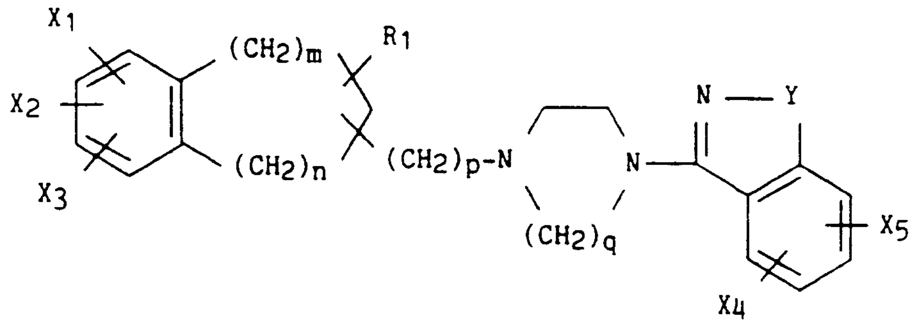

(I)

in which:
- $X_1$, $X_2$, $X_3$, $X_4$ and $X_5$:
  - which may be the same or different, each represent: a hydrogen atom, a halogen atom, an alkyl, alkoxy or alkylthio radical in each of which the alkyl moiety is straight-chained or branched and contains from 1 to 5 carbon atoms, a hydroxy radical, an acyloxy radical, a trifluoromethyl radical, a nitro radical, an amino radical or an acetamido radical, or
  - two of them in adjacent positions together form a methylenedioxy radical, an ethylenedioxy radical or a vinylenedioxy radical;
- $R_1$ represents a hydrogen atom or a straight-chained or branched alkyl radical containing from 1 to 5 carbon atoms;
- m and n each represent 0, 1, 2 or 3, provided that $m + n \geq 1$;
- p represents an integer of from 1 to 6;
- q represents 2 or 3; and
- Y represents an oxygen atom or the group $S(O)_z$ in which z is 0, 1 or 2;

in racemic and optically active forms.

2. The physiologically tolerable salts of the compounds of claim 1 with suitable acids.

3. A compound of claim 1 which is (R,S)-3-{1-[(benzocyclobutan-1-yl)methyl]piperazin-4-yl}-6-fluoro-1,2-benzisoxazole, and its hydrochloride.

4. A compound of claim 1 which is (R,S)-3-{1-[4-(benzocyclobutan-1-yl)butyl]piperazin-4-yl}-1,2-benzisothiazole, and its hydrochloride.

5. A compound of claim 1 which is (R,S)-3-{1-[(benzocyclobutan-1-yl)methyl]piperazin-4-yl}-1,2-benzisoxazole, and its hydrochloride.

6. A compound of claim 1 which is (R,S)-3-{1-[3-(benzocyclobutan-1-yl)propyl]piperazin-4-yl}-1,2-benzisothiazole, and its hydrochloride.

7. A compound of claim 1 which is 3-{1-[(5,6-dimethoxyindan-2-yl)methyl]piperazin-4-yl}-1,2-benzisothiazole.

8. A compound of claim 1 which is (R,S)-3-{1-[(4,5-dimethoxybenzocyclobutan-1-yl)methyl]piperazin-4-yl}-6-fluoro-1,2-benzisoxazole.

9. A compound of claim 1 which is (R,S)-3-{1-[(4,5-dimethoxybenzocyclobutan-1-yl)methyl]piperazin-4-yl}-1,2-benzisoxazole.

10. Process for the preparation of the compounds of claim 1, characterised in that:

- a compound of the general formula II:

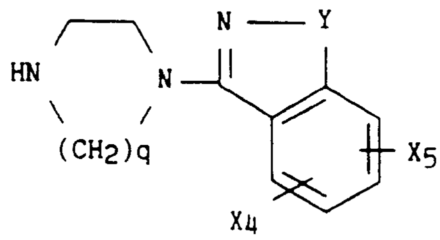

(II),

in which q, Y, $X_4$ and $X_5$ have the meanings defined in claim 1, is condensed with

- a compound of the general formula III:

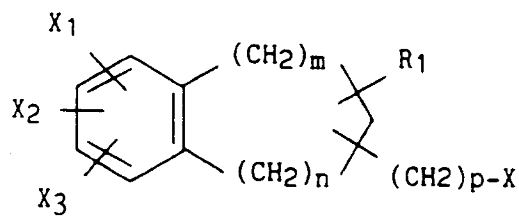

(III),

in which:

- $X_1$, $X_2$, $X_3$, $R_1$, m, n and p have the meanings defined in claim 1, and
- X represents a halogen atom, a mesyloxy radical or a tosyloxy radical.

**11.** Preparation process according to claim 10, characterised in that the condensation of the compounds II and III is carried out in a suitable solvent at a temperature of from 20 to 150 °C, in the presence of an acceptor for the acid formed during the reaction.

**12.** Process for the preparation of the compounds of claim 1, characterised in that:

- a compound of the general formula II defined in claim 10 is condensed with
- a compound of the general formula IV:

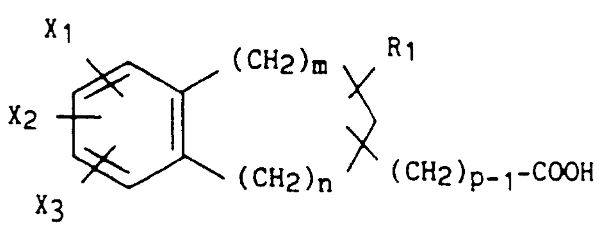

(IV),

in which $X_1$, $X_2$, $X_3$, $X_4$, $X_5$, $R_1$, m, n and p have the meanings defined in claim 1; and

- the resulting amide of the general formula V:

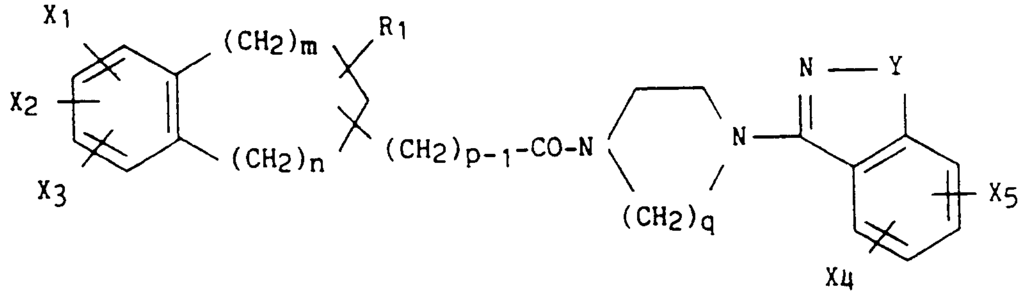

(V),

in which $X_1$, $X_2$, $X_3$, $X_4$, $X_5$, $R_1$, m, n, p, q and Y have the meanings defined above, is reduced.

**13.** Preparation process according to claim 12, characterised in that the condensation of the compounds II and IV is carried out in methylene chloride in the presence of carbonyldiimidazole.

**14.** Preparation process according to claims 12 and 13, characterised in that the amide V is reduced by means of a double hydride of lithium and aluminium in a suitable solvent.

**15.** As intermediates that can be used in the synthesis of the compounds I according to claim 12, the amides of the general formula V:

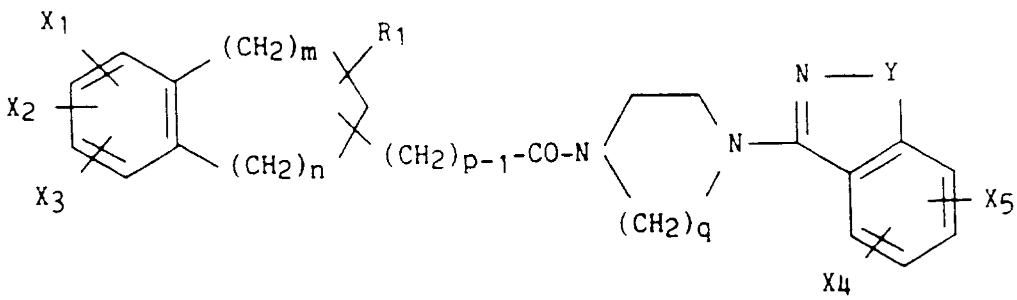

(V)

in which $X_1$, $X_2$, $X_3$, $X_4$, $X_5$, $R_1$, m, n, p, q and Y have the meanings defined in claim 1.

**16.** Pharmaceutical compositions containing as active ingredient a compound according to claims 1 to 10, together with suitable pharmaceutical excipients.

**17.** Pharmaceutical compositions according to claim 16 in a form suitable in particular for the treatment of pain, anxiety, psychosis, schizophrenia and depression.

**Claims for the following Contracting States : ES, GR**

**1.** Process for the preparation of benzisoxazole and benzisothiazole compounds of the general formula I:

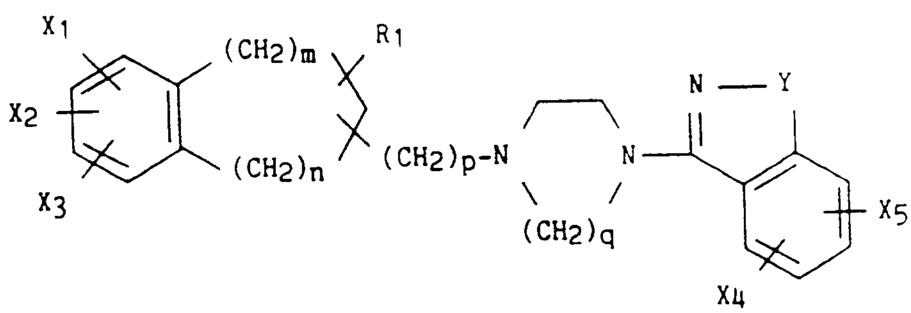

(I)

in which:
- $X_1$, $X_2$, $X_3$, $X_4$ and $X_5$:
  - which may be the same or different, each represent: a hydrogen atom, a halogen atom, an alkyl, alkoxy or alkylthio radical in each of which the alkyl moiety is straight-chained or branched and contains from 1 to 5 carbon atoms, a hydroxy radical, an acyloxy radical, a trifluoromethyl radical, a nitro radical, an amino radical or an acetamido radical, or
  - two of them in adjacent positions together form a methylenedioxy radical, an ethylenedioxy radical or a vinylenedioxy radical;
- $R_1$ represents a hydrogen atom or a straight-chained or branched alkyl radical containing from 1 to 5 carbon atoms;
- m and n each represent 0, 1, 2 or 3, provided that $m + n \geq 1$;

- p represents an integer of from 1 to 6;
- q represents 2 or 3; and
- Y represents an oxygen atom or the group $S(O)_z$ in which z is 0, 1 or 2;

in racemic and optically active forms, and of their physiologically tolerable acid addition salts, characterised in that:
- a compound of the general formula II:

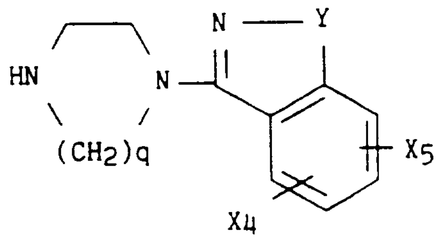

(II),

in which q, Y, $X_4$ and $X_5$ have the meanings defined above, is condensed with
- a compound of the general formula III:

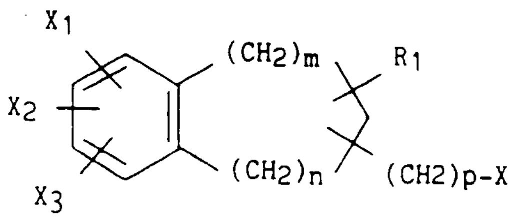

(III),

in which:
- $X_1$, $X_2$, $X_3$, $R_1$, m, n and p have the meanings defined above, and
- X represents a halogen atom, a mesyloxy radical or a tosyloxy radical;

and, if desired, the compound so obtained is treated with suitable acids to yield the corresponding acid addition salts.

**2.** Preparation process according to claim 1, characterised in that the condensation of the compounds II and III is carried out in a suitable solvent at a temperature of from 20 to 150 °C, in the presence of an acceptor for the acid formed during the reaction.

**3.** Process for the preparation of the compounds of formula I defined in claim 1, characterised in that:
- a compound of the general formula II defined in claim 1 is condensed with
- a compound of the general formula IV:

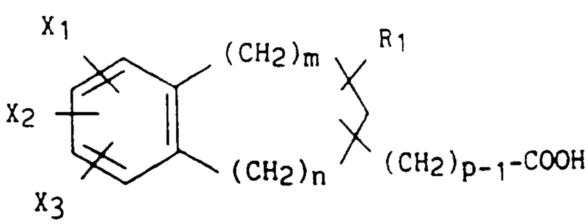

(IV),

in which $X_1$, $X_2$, $X_3$, $X_4$, $X_5$, $R_1$, m, n and p have the meanings defined in claim 1; and

- the resulting amide of the general formula V:

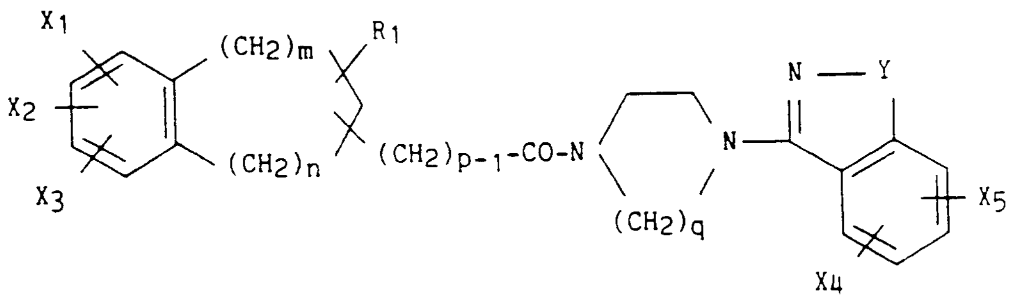

(V),

in which $X_1$, $X_2$, $X_3$, $X_4$, $X_5$, $R_1$, m, n, p, q and Y have the meanings defined above, is reduced; and, if desired, the compound so obtained is treated with suitable acids to yield the corresponding acid addition salts.

4. Preparation process according to claim 3, characterised in that the condensation of the compounds II and IV is carried out in methylene chloride in the presence of carbonyldiimidazole.

5. Preparation process according to claims 3 and 4, characterised in that the amide V is reduced by means of a double hydride of lithium and aluminium in a suitable solvent.

**Patentansprüche**

**Patentansprüche für folgende Vertragsstaaten : AT, BE, CH, DE, DK, FR, GB, IT, LI, LU, NL, PT, SE**

1. Benzisoxazolderivate und Benzisothiazolderivate der allgemeinen Formel I:

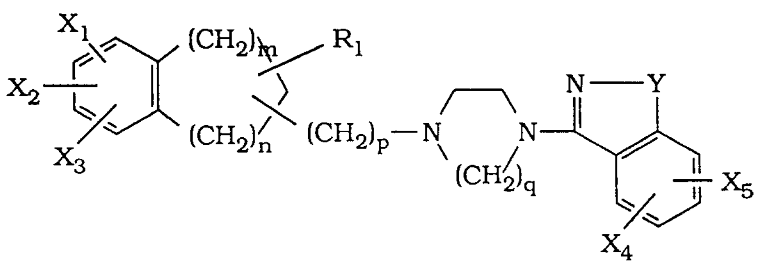

(I)

in der:
- $X_1$, $X_2$, $X_3$, $X_4$ und $X_5$:
  - die gleichartig oder verschieden sein können, jeweils ein Wasserstoffatom. ein Halogenatom, eine Alkyl-, Alkoxy- oder Alkylthiogruppe, worin jeder der Alkylreste jeweils 1 bis 5 Kohlenstoffatome in gerader oder verzweigter Kette aufweist, eine Hydroxylgruppe, eine Acyloxygruppe, eine Trifluormethylgruppe, eine Nitrogruppe, eine Aminogruppe oder eine Acetamidogruppe. oder
  - zwei dieser Gruppen in benachbarter Stellung gemeinsam eine Methylendioxygruppe, eine Ethylendioxygruppe oder eine Vinylendioxygruppe;
- $R_1$ ein Wasserstoffatom oder eine Alkylgruppe mit 1 bis 5 Kohlenstoffatomen in gerader oder verzweigter Kette;
- m und n jeweils die Werte Null, Eins, Zwei oder Drei mit der Maßgabe, daß $m + n \geqq 1$ ist;
- p eine ganze Zahl mit einem Wert von 1 bis 6;
- q Zwei oder Drei und
- Y ein Sauerstoffatom oder die Gruppe $S(O)_z$, worin z die Werte Null, Eins oder Zwei annehmen kann, bedeuten;

in racemischer oder optisch aktiver Form.

2. Die physiologisch verträglichen Salze der Derivate nach Anspruch 1 mit geeigneten Säuren.

**3.** Derivat nach Anspruch 1, nämlich (R,S)-3-{1-[(Benzocyclobutan-1-yl)-methyl]-piperazin-4-yl}-6-fluor-1,2-benzisoxazol und dessen Hydrochlorid.

**4.** Derivat nach Anspruch 1, nämlich (R,S)-3-{1-[4-(Benzocyclobutan-1-yl)-butyl]-piperazin-4-yl}-1,2-benzisothiazol und dessen Hydrochlorid.

**5.** Derivat nach Anspruch 1, nämlich (R,S)-3-{1-[(Benzocyclobutan-1-yl)-methyl]-piperazin-4-yl}-1,2-benzisoxazol und dessen Hydrochlorid.

**6.** Derivat nach Anspruch 1, nämlich (R,S)-3-{1-[3-(Benzocyclobutan-1-yl)-propyl]-piperazin-4-yl}-1,2-benzisothiazol und dessen Hydrochlorid.

**7.** Derivat nach Anspruch 1, nämlich 3-{1-[(5,6-Dimethoxyindan-2-yl)-methyl]-piperazin-4-yl}-1,2-benzisothiazol.

**8.** Derivat nach Anspruch 1, nämlich (R,S)-3-{1-[4,5-(Dimethoxybenzocyclobutan-1-yl)-methyl]-piperazin-4-yl}-6-fluor-1,2-benzisoxazol.

**9.** Derivat nach Anspruch 1, nämlich (R,S)-3-{1-[4,5-(Dimethoxybenzocyclobutan-1-yl)-methyl]-piperazin-4-yl}-1,2-benzisoxazol.

**10.** Verfahren zur Herstellung der Derivate nach Anspruch 1, **dadurch gekennzeichnet,** daß man
- ein Derivat der allgemeinen Formel II:

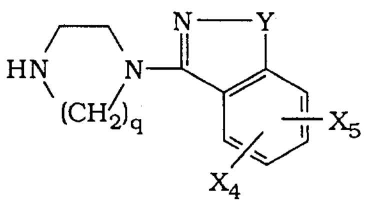

(II)

in der q, Y, $X_4$ und $X_5$ die in Anspruch 1 angegebenen Bedeutungen besitzen, mit
- einem Derivat der allgemeinen Formel III:

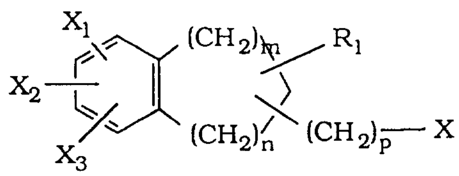

(III)

in der:
- $X_1$, $X_2$, $X_3$, $R_1$, m, n und p die in Anspruch 1 angegebenen Bedeutungen besitzen und
- X ein Halogenatom, eine Mesyloxygruppe oder eine Tosyloxygruppe darstellt,

kondensiert.

**11.** Verfahren nach Anspruch 10, **dadurch gekennzeichnet,** daß man die Kondensation der Derivate II und III in einem geeigneten Lösungsmittel bei einer Temperatur zwischen 20 und 150 °C in Gegenwart eines Akzeptors für die im Verlaufe der Reaktion gebildete Säure bewirkt.

**12.** Verfahren zur Herstellung der Derivate nach Anspuch 1, **dadurch gekennzeichnet,** daß man
- ein Derivat der in Anspruch 10 definierten allgemeinen Formel II mit

- einem Derivat der allgemeinen Formel IV:

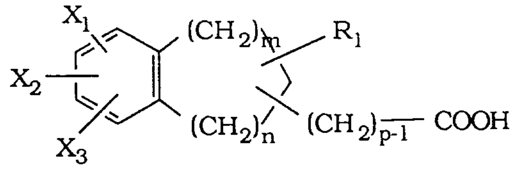

(IV)

in der $X_1$, $X_2$, $X_3$, $X_4$, $X_5$, $R_1$, m, n und p die in Anspruch 1 angegebenen Bedeutungen besitzen, kondensiert; und

- das in dieser Weise erhaltene Amid der allgemeinen Formel V:

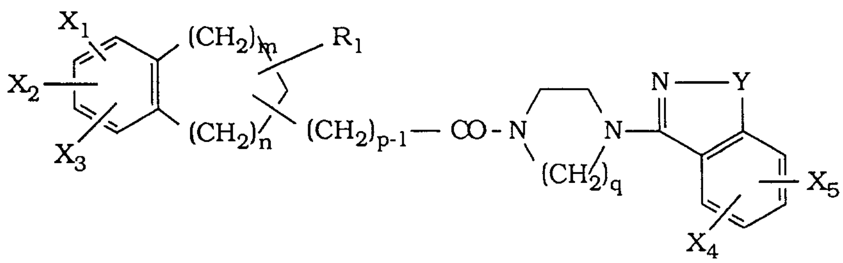

(V)

in der $X_1$, $X_2$, $X_3$, $X_4$, $X_5$, $R_1$, m, n, p, q und Y die oben angegebenen Bedeutungen besitzen, reduziert.

**13.** Verfahren nach Anspruch 12, **dadurch gekennzeichnet,** daß man die Kondensation der Derivate II und IV in Methylenchlorid in Gegenwart von Carbonyldiimidazol bewirkt.

**14.** Verfahren nach den Ansprüchen 12 und 13, **dadurch gekennzeichnet,** daß man die Reduktion des Amids V mit einem Lithiumaluminiumdoppelhydrid in einem geeigneten Lösungsmittel durchführt.

**15.** Als Zwischenprodukte für die Synthese der Derivate I nach Anspruch 12 geeignete Amide der allgemeinen Formel V:

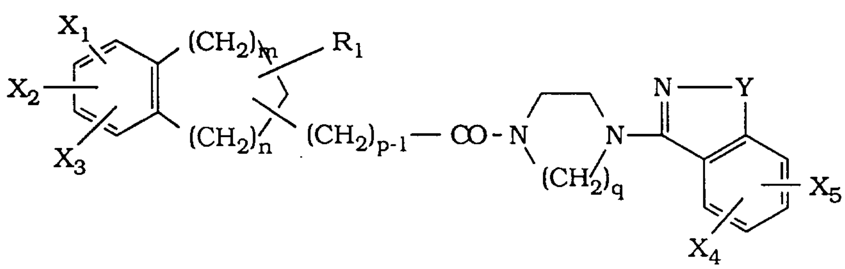

(V)

in der $X_1$, $X_2$, $X_3$, $X_4$. $X_5$, $R_1$, m, n, p, q und Y die in Anspruch 1 angegebenen Bedeutungen besitzen.

**16.** Pharmazeutische Zubereitungen enthaltend als Wirkstoff ein Derivat nach den Ansprüchen 1 bis 10 zusammen mit geeigneten pharmazeutischen Trägermaterialien.

**17.** Pharmazeutische Zubereitungen nach Anspruch 16 in einer Form, die insbesondere zur Behandlung von Schmerzen, der Angst, von Psychosen, der Schizophrenie und von Depressionen geeignet ist.

**Patentansprüche für folgende Vertragsstaaten : ES, GR**

1. Verfahren zur Herstellung der Benzisoxazol- und Benzisothiazolderivate der allgemeinen Formel I:

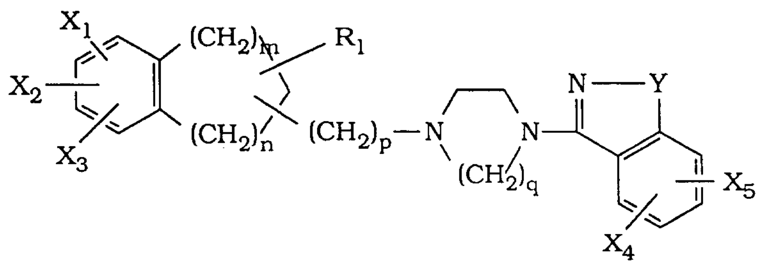

(I)

in der:

- $X_1$, $X_2$, $X_3$, $X_4$ und $X_5$:
  - die gleichartig oder verschieden sein können, jeweils ein Wasserstoffatom, ein Halogenatom, eine Alkyl-, Alkoxy- oder Alkylthiogruppe, worin jeder der Alkylreste jeweils 1 bis 5 Kohlenstoffatome in gerader oder verzweigter Kette aufweist, eine Hydroxylgruppe, eine Acyloxygruppe, eine Trifluormethylgruppe, eine Nitrogruppe, eine Aminogruppe oder eine Acetamidogruppe, oder
  - zwei dieser Gruppen in benachbarter Stellung gemeinsam eine Methylendioxygruppe, eine Ethylendioxygruppe oder eine Vinylendioxygruppe;
- $R_1$ ein Wasserstoffatom oder eine Alkylgruppe mit 1 bis 5 Kohlenstoffatomen in gerader oder verzweigter Kette;
- m und n jeweils die Werte Null, Eins, Zwei oder Drei mit der Maßgabe, daß $m + n \geqq 1$ ist;
- p eine ganze Zahl mit einem Wert von 1 bis 6;
- q Zwei oder Drei und
- Y ein Sauerstoffatom oder die Gruppe $S(O)_z$, worin z die Werte Null, Eins oder Zwei annehmen kann, bedeuten;

in racemischer oder optisch aktiver Form, sowie von deren physiologisch verträglichen Säureadditionssalzen, **dadurch gekennzeichnet,** daß man

- ein Derivat der allgemeinen Formel II:

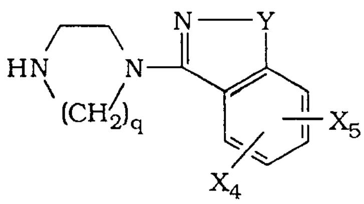

(II)

in der q, Y, $X_4$ und $X_5$ die oben angegebenen Bedeutungen besitzen, mit

- einem Derivat der allgemeinen Formel III:

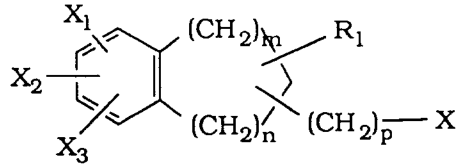

(III)

in der:

- $X_1$, $X_2$, $X_3$, $R_1$, m, n und p die oben angegebenen Bedeutungen besitzen und
- X ein Halogenatom, eine Mesyloxygruppe oder eine Tosyloxygruppe darstellt, kondensiert,

und gewünschtenfalls das in dieser Weise erhaltene Derivat zur Bildung der entsprechenden Säureadditionssalze mit geeigneten Säuren behandelt.

2. Verfahren nach Anspruch 1, **dadurch gekennzeichnet,** daß man die Kondensation der Derivate II und III in einem geeigneten Lösungsmittel bei einer Temperatur zwischen 20 und 150 °C in Gegenwart eines Akzeptors für die im Verlaufe der Reaktion gebildete Säure bewirkt.

3. Verfahren zur Herstellung der Derivate der Formel I, wie sie in Anspruch 1 definiert ist, **dadurch gekennzeichnet,** daß man
   - ein Derivat der in Anspruch 1 definierten allgemeinen Formel II mit
     - einem Derivat der allgemeinen Formel IV:

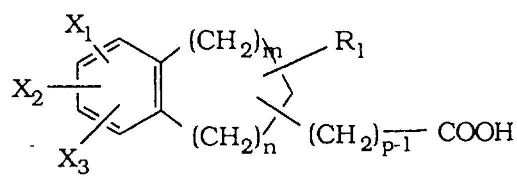

(IV)

in der $X_1$, $X_2$, $X_3$, $X_4$, $X_5$, $R_1$, m, n und p die in Anspruch 1 angegebenen Bedeutungen besitzen, kondensiert; und
     - das in dieser Weise erhaltene Amid der allgemeinen Formel V:

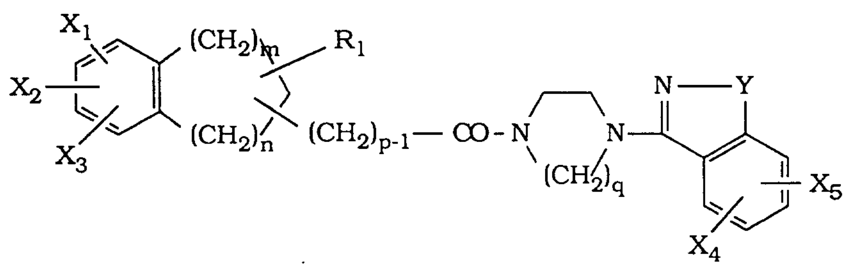

(V)

in der $X_1$, $X_2$, $X_3$, $X_4$, $X_5$, $R_1$, m, n, p, q und Y die oben angegebenen Bedeutungen besitzen, reduziert;
und gewünschtenfalls das in dieser Weise erhaltene Derivat zur Bildung der entsprechenden Säureadditionssalze mit geeigneten Säuren behandelt.

4. Verfahren nach Anspruch 3, **dadurch gekennzeichnet,** daß man die Kondensation der Derivate II und IV in Methylenchlorid in Gegenwart von Carbonyldiimidazol bewirkt.

5. Verfahren nach Anspruch 3 und 4, **dadurch gekennzeichnet,** daß man die Reduktion des Amids V mit einem Lithiumaluminiumdoppelhydrid in einem geeigneten Lösungsmittel durchführt.